# EUROPEAN PATENT SPECIFICATION

(11) **EP 1 062 840 B1**
(45) Date of publication and mention of the grant of the patent: **29.03.2006**
(21) Application number: 98966028.7
(22) Date of filing: 18.12.1998
(51) Int. Cl.: H04R 25/00

(54) **APPARATUS AND METHOD FOR A CUSTOM SOFT-SOLID HEARING AID**
VORRICHTUNG UND VERFAHREN FÜR PASSBARES WEICHES FESTES HÖRHILFEGERÄT
APPAREIL ET PROCEDE DESTINES A UNE PROTHESE AUDITIVE SOUPLE/SOLIDE

(30) Priority: 18.12.1997 US 68035 P; 26.05.1998 US 84864; 28.10.1998 US 181539; 28.10.1998 US 181540; 28.10.1998 US 181541; 28.10.1998 US 181842; 28.10.1998 US 181843; 28.10.1998 US 181844; 28.10.1998 US 181845
(43) Date of publication of application: 27.12.2000
(73) Proprietor: Softear Technologies, L.L.C., Harahan, LA 70123 (US)
(72) Inventor: JUNEAU, Roger, P., Destrehan, LA 70047 (US); CREEL, Lynn, P., Kenner, LA 70065 (US); DESPORTE, Edward, J., Covington, LA 70433 (US); MAJOR, Michael, W., Kenner, LA 70065 (US); SIEGLE, Gregory, R., Kenner, LA 70065 (US); KINLER, Kelly, M., Luling, LA 70070 (US)
(74) Representative: Harrison Goddard Foote
(86) International application number: PCT/US1998/027113
(87) International publication number: WO 1999/031935

(56) References cited:
- WO-A-93/25053
- DE-U- 29 608 215
- JP-A- 61 238 198
- US-A- 4 375 016
- US-A- 4 617 429
- US-A- 4 962 537
- PATENT ABSTRACTS OF JAPAN vol. 016, no. 475 (E-1273), 2 October 1992 (1992-10-02) -& JP 04 170899 A (TERUMO CORP), 18 June 1992 (1992-06-18)

## Description

### ASSIGNEE

SOFTEAR TECHNOLOGIES, L.L.C. (a Louisiana, US, limited liability company), 175 Brookhollow Esplanade, Harahan, LA 70123, US.

### SPECIFICATION

### CROSS-REFERENCE TO RELATED APPLICATIONS

In the US, this is a continuation-in-part of our co-pending US Patent Application Serial Nos. 09/181,539,09/181,540,09/181/541,09/181/842,09/181/843, 09/181/844, and 09/181/845, all filed 28 October 1998, which are continuations-in-part of our co-pending US Patent Application Serial No. 09/084,864, filed 26 May 1998. Priority of these applications is hereby claimed.

Priority of US Provisional Patent Application Serial No. 60/068,035, filed 18 December 1997, incorporated herein by reference, is hereby claimed.

### STATEMENT REGARDING FEDERALLY SPONSORED RESEARCH OR DEVELOPMENT

Not applicable

### REFERENCE TO A "MICROFICHE APPENDIX"

Not applicable

### BACKGROUND OF THE INVENTION

### I. Field of the Invention

The present invention relates to an ear-worn device that is comprised of a soft yet solid elastomer corpus for use in custom in-the-ear hearing products. The degree of stiffness of this soft-solid material preferably ranges from negligible to forty points, Durometer Hardness, Shore A. Specifically, the present invention relates to a system and method for producing a custom soft yet solid elastomer hearing product yielding greater comfort and superior acoustic performance for the hearing instrument wearer. Additionally, this product will provide solutions to a population with whom traditional custom in-the-ear technology was unsuccessful. By the nature of its soft design this product will have improved compliance and elasticity, thereby better accommodating the dynamic nature and the anatomical variants of the external ear canal. This invention will also relate to future applications in the field of mass communications, such as an ear-worn digital telephone or a two-way radio system.

### II. General Background of the Invention

The Hearing Instrument Industry combines electro-acoustic technology with custom prosthetic design to ergonomically couple a hearing instrument to the human ear in a cosmetically acceptable manner. The industry has realized major electronic advancements in hearing instrument technology. With miniaturization of electronic components the standard instrument design evolved from a table worn unit, using vacuum tubes in the nineteen twenties, to a wearable body worn unit in the late nineteen thirties. The introduction of transistors in the nineteen fifties made the behind-the-ear (BTE) hearing instrument possible. As integrated circuits were developed, the custom in-the-ear (ITE) instrument became a reality. On-going electronic developments surrounding the hearing instrument industry have resulted in the microminiaturization of electronic components. This miniaturization has culminated in the introduction of "deep insertion technology", manifested as the completely-in-the-canal (CIC) hearing instrument, which is totally contained within the ear canal and is virtually invisible. As a consequence, hearing instruments have increased signal processing capabilities, yet require very limited physical space.

With the development of programmable hearing instruments, using either analog or digital signal processing, custom electronic design has shifted from the manufacturing level to the clinical level. That is, the clinician can now customize the electro-acoustic response of the instrument to match the degree of hearing loss via programmable software. It is no longer necessary for the device to be returned to the manufacturer for hardware changes to achieve the desired electro-acoustic characteristics.

In direct contrast to electronic advances within the industry, little or no advancement has been realized in custom prosthetic design. Since the late nineteen sixties, when the custom instruments were developed, the materials and the construction techniques have remained virtually unchanged. These materials and techniques were adopted from the dental industry, whereby the customized housing- commonly called a "shell"- was constructed using acrylic with a ninety point "D" Shore Hardness. Typical molding of the dental acrylate involves making a female silicone cavity from the original ear impression. This female cavity is then filled with liquid acrylate and cured using an ultraviolet light of known intensity across a known time period to cure only the outer most material forming a wall or a shell. This process is very similar to ceramics. The shell is then removed from the female cavity, decked down in the sagittal plane, drilled for vents and receiver bores, polished and then mounted with a faceplate containing the electro-acoustic circuitry. The end result is a hollow glass-like plastic replica of the external ear canal. The finished shell's primary function is to house the delicate electronic components. Yet, a material of this hardness, worn deeply in the human ear canal, brings forth the issues of comfort and acoustic performance.

When the acrylic shell was introduced, hearing instruments were worn in a relatively elastic cartilaginous portion of the ear canal. However, the current trend for hearing instrument placement is to position the device into the bony portion of the ear canal extending three millimeters medially from the second directional bend previously defined as "deep insertion technology". To illustrate the implications of this technology, the anatomy and physiology of the ear will be reviewed.

Anatomically, the ear canal is defined as the area extending from the concha to the tympanic membrane. It is important to note that the structure of this canal consists of elastic cartilage laterally, and porous bone medially covered by skin. The cartilaginous portion constitutes the outer one third of the ear canal. The medial two-thirds of the ear canal is osseous or bony and is oriented forward and downward making it slightly concave as compared to the more cylindrical cartilaginous portion. The average canal is approximately twenty-five millimeters in length but is as much as six millimeters longer on the anteroinferior wall of the osseous canal. The skin of the osseous canal, measuring only two-tenths of a millimeter (0.2 mm) in thickness, is much thinner than the skin of the cartilaginous canal, measuring five-tenths to one millimeter (0.5 to 1 mm) in thickness. The difference in thickness directly corresponds to the presence of apocrine (ceruminous) and sebaceous glands found only in the fibro-cartilaginous area of the canal. This thinly skinned, thinly lined area of the bony canal is extremely sensitive to any hard foreign body, such as an acrylic hearing instrument.

Physiologically, the ear canal is dynamic in nature. It is geometrically altered by mandibular action and by head position changes. These cause alternating elliptical elongation and widening of the ear canal. These alterations in canal shape vary widely, not only from person to person, but also from ear to ear.

Applying hard, hollow, acrylic hearing instrument technology to the external ear canal has numerous limitations. Because of the rigid nature of the acrylic shell of many traditional instruments, they are difficult to insert beyond the second directional canal bend. The difficulty of insertion is increased in the presence of any anatomical variant such as a stenotic canal, a bulbous canal, or a tortuous canal.

Because of the rigid nature of the acrylic shell of many traditional instruments, they must pivot in reaction to mandibular action or head movement, thereby changing the angle of attack of the receiver toward the tympanic membrane resulting in a distorted acoustic response.

Additionally, this pivoting action often causes displacement of the entire instrument causing a slit leak between the wall of the device and the wall of the ear canal. That leak creates an open acoustic loop between the receiver and the microphone of the instrument resulting in an electro-acoustic distortion commonly known as feedback.

Because of the rigid nature of the acrylic shell, some deeply inserted traditional instruments will exert pressure upon the bony portion of the ear canal when mandibular action or head movement cause the instrument to pivot.

Because of the hollow nature of the acrylic shell, many traditional instruments cannot protect the internal components from damage due to shock (i.e. the impact suffered by a traditional instrument dropped onto a hard surface).

Because of the hollow nature of the acrylic shell, many traditional instruments provide an air-conducted feedback loop from the receiver to the microphone.

Because of the hollow nature of the acrylic shell and the inherent necessity to suspend the receiver by tube mounting, the traditional instrument is prone to collection of cerumen in the receiver tube. Attempts to excavate the cerumen often breaks the bond between the receiver tube and the receiver port of the shell, displacing the receiver into the instrument.

Because of the solid nature of the acrylic shell, the proximal tip of many traditional instruments serves as a reverberating surface for acoustic energy reflected by the tympanic membrane resulting in distortion.

Because of the rigid and hollow nature of the acrylic shell, traditional instruments with such a shell have relatively widely separated interior surfaces that promote internal acoustic reverberation and its attendant feedback.

To compensate for these limitations, modification to the hard shell exterior to approximate the anatomical variants and to meet the demands of the dynamic nature of the ear canal are performed. The shell is buffed and polished until comfort is acceptable without significantly compromising acoustic performance. The peripheral acoustic leakage caused by these modifications often results in acoustic feedback (whistling) before sufficient amplification can be attained. Additionally, this acoustic leakage causes annoying low frequency sounds to be inadvertently amplified by means of a Helmholtz resonator. Patients commonly report this sensation as "My voice is hollow" or "My head sounds like it is in a barrel."

Another approach taken to compensate for the limitations of the hard, hollow acrylic shell has been to alter the electro-acoustic parameters of the instruments. It was expected that, with the development of programmable devices, a sophisticated, precise electro-acoustical method of eliminating these acoustic anomalies would be available to the clinician. Ironically, the improved frequency spectrum of the programmable instruments exacerbated the problem. The practical solution was to adjust the program of an instrument which was exhibiting feedback by reducing the high frequency information, or to retreat to a larger behind-the-ear hearing aid.

Faced with the limited success of shell or electro-acoustic modification, a few manufacturers have attempted all-soft shells. Wearers did report greater comfort and better sound quality. Unfortunately, while rigid acrylic does not lose its dimensional stability, soft vinyl materials shrink, discolor, and harden after a relatively short period of wear (the replacement of vinyl material used for BTE earmolds, for example, is recommended on at least a yearly basis). Polyurethane provides a better acoustic seal than polyvinyl, but has an even shorter wear life (approximately three months). Silicones have a long wear life but are difficult to bond to plastics, a necessary process for the construction of custom hearing instruments. Furthermore, silicone is difficult to modify when the dimensional structure requires alteration for proper fit. To date, then, acrylic has proven to be the only material with long term structural integrity. The fact remains, however, that the entire ear is a dynamic acoustic environment ill-served by a rigid material such as acrylic.

Some references of interest are discussed below.

U.S. Pat. No. 4,870,688 to Voroba, Barry, et al.

Voroba describes a patient selected mass produced, non-custom molded form fitting shell with a malleable covering having a hook and twist which in theory precisely conforms to the patient's own ear.

U.S. Pat. No. 4,880,076 to Ahlberg, Carl, et al.

Ahlberg discloses a user-disposable foam sleeve comprising a soft polymeric retarded recovery foam that can be compressed to be freely inserted into the patient's ear and then allowed to expand until secure in the ear canal.

Other patents that may be of interest include the following:
U.S. Pat. No. 5,002,151 to Oliveira, Robert J., et al.;
U.S. Patent No. 4,607,720 HEARING AID;
U.S. Patent No. 4,375,016 VENTED EAR TIP FOR HEARING AID AND ADAPTER COUPLER THEREFOR;
U.S. PatentNos.: 4,051,330; 4,716,985; 4,811,402; 4,937,876; 4,962,437 5,068,902; 5,185,802; 5,201,007; 5,259,032; 5,530,763; 5,430,801; 5,500,902; and 5,659,621, International Patent Nos. WO 93/25 053 and WO 92/03 894.

Also of interest are published Japanese patent application no. JA61-238198, the articles from December 1997 JOURNAL OF AMERICAN ACADEMY OF AUDIOLOGY, and Staab, Wayne J. and Barry Finlay, "A fitting rationale for deep fitting canal hearing instruments", HEARING INSTRUMENTS, Vol. 42, No. 1, 1991, pp. 7-10, 48.

### BRIEF SUMMARY OF THE INVENTION

The present invention relates generally to in-the-ear hearing aids and particularly to a soft elastomer solid within which the electronic components are embedded. The hearing aids can be custom or mass produced. The general objective of this invention is to provide a product which is authentic to the shape of the external ear canal, yet compliant enough to compensate for the dynamic properties of the canal. In addition to that general objective, there are specific objectives. This objective is accomplished by providing a body which, in the preferred embodiment of the present invention, has a 5-15 durometer, Shore A hardness to optimize the following apparently opposing desired characteristics:
the device should be stiff enough to easily insert into the ear;
the device should be soft enough to compress and recover, once inserted into the ear, as the jaw's mandibular action flexes the external ear canal changing the horizontal diameter as it opens and closes; and
the device should not tear or elongate during stretching to the point that the strain relief systems between the amplifier and receiver would be overly burdened to failure. i.e. wires or connections would not break when the device is flexed by tensile force, compression, torsion or a complex combination of the three forces.

The present invention includes an ear-worn hearing device a hearing device body sized and shaped to generally fit into a human ear canal; and an electronic hearing circuit embedded in the body, wherein the body is non hollow, is made of a soft-solid made of an elastomer and has a Durometer Hardness, Shore A, of less than 40 points.

The body of the device of the present invention preferably comprises an elastomer blend which once completely vulcanized is bondable to another elastomer blend which has not been vulcanized and which is of similar elastomeric characteristics in the finished and unfinished state, both of which can be formulated at the very low end of the shore A scale (preferably ranging from 0-17 Durometer). This bonding capability will provide a platform for the resizing of an existing device and further provide the platform for the manufacture of a series of universal, non-custom, soft-solid devices. These universal devices could be customized by the manufacturer or at the clinic on a later date using a dipping process or some other external patching technique to bond the existing soft-solid device to a new outer layer of soft-solid elastomer.

This bonding capability will provide the platform for the manufacture of a soft-solid device of two different designs. The first design would be similar to an M&M brand peanut candy, where the electronic components are the peanut. The inside chocolate would be made of elastomers which are stiff (27 Shore A Durometer, e.g.) to serve as a skeleton support for insertion into the ear. This center medium would be relatively thin compared to the candy, say 2mm or less. The outer candy shell would be very soft (e.g. 7-10 Shore A Durometer) and would be thick, 3mm or more as the size dictates.

The second design would be more like the candy model, in that the center would be very soft elastomer (3-10 Shore A Durometer, e.g.) and approximately 3-4mm in thickness as the ear canal allows and outer shell would be approximately 27 Shore A Durometer, e.g., and for example 1 mm thick. In this design the insertion rigidity would be provided by the outer layer. The components in the inner layer would be embedded in soft elastomer allowing it to move more easily and would not bond the components as tightly reducing the conductive pathways between the receiver and other components.

Both of these approaches would allow the product to be inserted into the ear, maintain wall pressure on the ear canal wall, and allow the components to move with dynamic motion with the aid of a proper wire stain relief system described later herein.

The present invention, because of its soft nature, will not migrate out of the external ear canal with jaw excursion. It is resistant to the lateral migration which is innate to traditional non-compliant shell.

The present invention, by its soft nature, will remain authentic to the topography of the ear canal, and will remain acoustically sealed with jaw excursion. The acoustic seal will reduce the peripheral leakage, and allow for greater gain and sound pressure before feedback. Concomitant with the improved acoustic seal and the elimination of the slit leak, there will be greater mid-frequency amplification and the elimination of high frequency roll-off, thereby emphasizing those frequencies most important for the perception of consonantal cues.

A total of 24 patients (44 ears) have been fit with the soft-solid device for the purpose of pilot investigations and product development. Results of these pilot investigations have shown the device to be more comfortable when compared with standard acrylic instruments. The soft-solid instrument was shown to accommodate the pivotal action of the jaw in that most wearers reported that the hearing aid, once seated, did not need repositioning over the course of the day. In other words, this soft-solid material was found to eliminate the migration of the hearing aid from the ear due to jaw movement. Hearing instrument users with a history of excessive feedback reported a reduction in feedback when using the soft-solid devices. For some wearers, feedback was entirely eliminated when using a soft-solid device. Finally, pilot investigations found the soft-solid instrument appropriate for patients with bulbous, tortuous, or surgically altered ear canals.

In addition to the improvements in fit and comfort, the soft-solid instruments were found to provide more overall gain in the ear canal than acrylic devices due to the reduction of feedback. In other words, patients were able to increase the gain of the hearing aid (via the volume control) more in the soft-solid devices before reaching the point of feedback. This increased utility of gain may allow for a greater fitting range of completely-in-the-canal hearing instruments. Finally, many patients reported improved sound quality and "distinctness of sounds" when comparing the soft-solid devices to acrylic devices. This may be attributed to the finding in the pilot investigations that the soft-solid devices produced greater mid-frequency gain in the real ear compared to an acrylic device with the same 2cc data.

These preliminary finding of the pilot investigation warrant a controlled clinical trial of the soft-solid hearing device.

The present invention, because of its soft nature, will not exert pressure on the bony portion of the external ear canal making it easily insertable beyond the second anatomical bend. This deeper insertion reduces residual volume between the proximal tip of the instrument and the tympanic membrane. Because sound pressure increases as residual volume decreases, more power is perceived without a corresponding increase in the gain of the instrument.

The present invention, because of its soft nature, will better accommodate anatomic aberrations such as tortuous ear canals, bulbous ear canals, stenotic ear canals, and iatrogenically altered ear canals.

The present invention, because of its solid nature, will protect the embedded electronic components.

The present invention, because of its solid nature, will eliminate the internal air conducted feedback pathway from the receiver to the microphone.

The present invention, because of its solid nature, will eliminate the need to suspend the receiver by tube-mounting, thereby preventing displacement of the receiver within the hearing instrument and eliminating the concomitant Helmholz resonation.

The present invention, because of its soft-solid nature, can be formulated in such a way as that the elastomer can be blended with conductive particles, such as gold dust or ferrite dust, to form a static shield protecting the circuitry from Radio Frequency Interference (RFI), Global System for Mobile Communication (GSM), and Electromagnetic Interference (EMI).

The present invention, because of its solid nature, can support the receiver and associated tubing. Because of its soft nature, the invention is compressible. Therefore, by compressing the tip of the instrument, cerumen can be extruded away from the receiver and out of the receiver port.

The present invention, because of its soft proximal tip, has a less reflective external surface than the traditional acrylic tip, thereby reducing intermodulation and reverberation.

The present invention, because of its solid nature, has no internal reflective surfaces, thereby eliminating internal reflection and reverberation.

The present invention, preferably comprising an elastomer blend at the very low end of the shore A scale (preferably ranging from 0-17 Durometer), will provide a platform for the manufacture of a series of universal, non-custom, soft-solid devices.

The present invention, because of its solid nature, provides for precise, uniform vent diameters, thereby providing predictable electro-acoustic responses.

The present invention, when incorporating a soft faceplate, results in a completely soft hearing instrument.

The present invention, because it is processed by casting a female cavity from the impression, eliminates buffing, waxing, and other means of impression modification inherent to current shell manufacturing procedures. By streamlining the assembly procedure, the present invention is more easy to produce and--consequently--less expensive to manufacture than the traditional hard, hollow acrylic shell.

The present invention includes the soft-solid body described herein, even when it is not filled with electronic components.

The present invention will accommodate additional personal communication devices such as telephones, pagers, memo-recorders, and two-way communication devices, instead of or in addition to hearing aids. In some cases (especially when a hearing aid is not included) it will be desirable to not make a good acoustic seal so that the person using the personal communication devices can also easily hear what is around him.

The present invention, because of its solid nature, can accommodate a bladder (preferably mounted in the center) which can be filled with a soft material while positioned within the human ear canal. While the device remains in the ear canal, the soft center can be allowed to cure to a hardness less than 35 Durometer Hardness, Shore A. Also, the bladder could be used to vary the amount of occlusion by varying the amount of gel present in the bladder - in such a case, there could be a valve to allow filling and evacuation of the bladder. A hypodermic syringe, for example, could be used to fill and evacuate the bladder. The soft material can comprise a gel elastomer or petroleum jelly, for example.

Also, a bladder could act as the outer lining which contacts the human ear inside which bladder all or substantially all of the electronic components are encapsulated. The volume of the bladder can be varied as in the above paragraph.

The present invention can be produced with a sufficiently low Durometer (e.g., 3-17) so as to allow sleeping with the instrument in place.

The current preferred embodiment of the invention eliminates the need for modifications of ear impressions, generally accomplished through altering the shape of the impression and waxing the altered impression. Hence, a more direct method of casting is achieved, yielding greater accuracy of topographical detail of the ear canal in terms of dimensional and geometric characteristics.

The preferred embodiment utilizes a material that is a blended silicone that accepts bonding to plastic by adhesives. Other embodiments of the invention may utilize rubbers, elastomers, and other rubber-like materials including neoprene, silicone, vinyl, butyl and soft plastics.

Preferably, the body occupies at least 70% of the volume of the hearing device not occupied by the electronic hearing circuit. More preferably, the body occupies at least 80% of the volume of the hearing device not occupied by the electronic hearing circuit. Even more preferably, the body occupies at least 90% of the volume of the hearing device not occupied by the electronic hearing circuit. Most preferably, the body occupies at least 99% of the volume of the hearing device not occupied by the electronic hearing circuit.

The outer surface of the body of the present invention is preferably non-absorbent and virtually impervious to cerumen.

The present invention is more fully described in the claims as filed in the parent patent applications and in the present application as filed, all of which claims are incorporated by reference.

### BRIEF DESCRIPTION OF THE SEVERAL VIEWS OF THE DRAWINGS

The manufacturing method according to the invention and the hearing instrument manufactured by this method is further described in the following detailed drawings, wherein:
FIG. 1 shows the device of the preferred embodiment of the present invention in a typical human ear from a side perspective;
FIG. 2 shows the device in a graphically illustrated view of a typical human ear viewed superiorly.
FIG. 3 shows the device as described in FIG. 2 except with an anatomic variant defined as a bulbous ear canal;
FIG. 4 shows the device as described in FIG. 2 except with an anatomic variant defined as a stenotic ear canal;
FIG. 5 shows the device as described in FIG. 2 except with an anatomic variant defined as a tortuous ear canal;
FIG. 6 shows the device as described in FIG. 2 except with an iatrogenic variant demonstrating a post surgical ear canal;
FIG. 7 shows the device as described in FIG. 2 demonstrating ear canal configuration change and canal angle variance between the jaw open position and the jaw closed position.
FIG.8 shows preferred embodiment of the invention including one embodiment of the wax chamber;
FIG.9 shows an acoustic illustration of a typical response;
FIGS. 10 and 11 show two typical hearing aid designs being currently produced;
FIG. 12 shows an alternative tubed wax guard system;
FIG. 13 shows an alternative capacitor location to reduce heterodyning of the amplified signal;
FIG. 14 shows an embodiment of the strain relief system of the present invention;
FIGS. 15-17 show a strain relief and receiver positioning system of the preferred embodiment of the present invention; and
FIG. 18 shows a microphone positioning and assembly system which can be used with traditional hearing aids as well as with the hearing aid of the present invention.

### DETAILED DESCRIPTION OF THE INVENTION

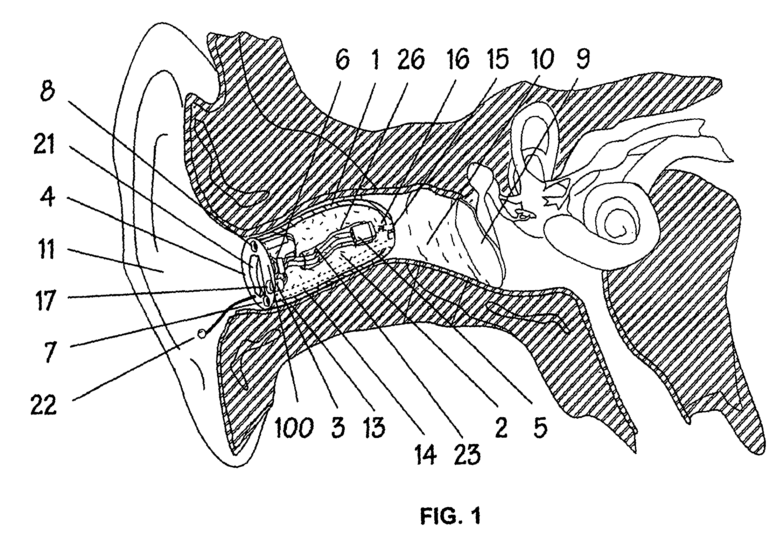

FIG. 1 shows one configuration of a complete device in the human ear from a side perspective. Outer layer 1 is a soft outer layer (such as a polydimethysilioxane layer of 35 Durometer hardness shore A, such as Dow Corning® MDX4-4210 (which is used in the current embodiment)). This outer layer 1 acts as a general surface skin that provides greater resistance to tearing and provides the stiffness for insertion. The outer layer 1 also provides resilience, durability, and abrasion resistance. A softer center 2 is preferably made of the same polydimethysilioxane except with functional groups and fillers (such as Intrinsic II (1300) and Functional Fluid (A313), and is preferably less than seventeen points Durometer hardness, shore A, such as Factor II Realistic (A-588). This soft center provides the wall pressure necessary to maintain the acoustic seal between the device 100 and the ear canal wall 13 from the sagittal plane of the aperture medially to the end of the device. Further, the composite of outer skin 1 and center 2 has elastomeric properties that allow the device to flex with jaw excursion, thereby eliminating the external acoustic loop between the receiver 5 and the microphone 8.

Layers 1 and 2 can be of equal stiffness and can be only a single homogeneous filling composing the center and outer boundaries and everything in between. Thus, the device body could be homogenous (the outer layer could be eliminated). It is believed that the flexibility of viewing these as separate layers allows for the mixing and refining of different elastomeric characteristics.

In one embodiment, a ratio of elastomer, non-functional extenders and catalyst is used in layer 2 with the final hardness of layer 2, the center layer, being off the A scale, i.e. "0" A or an elastomer gel. The end result is a soft, gel filled device which is compliant and flows with pressure. This compliance serves to eliminate the pivoting of the device that results in displacement. The soft center also serves to eliminate the internal air conduction path between the receiver 5 and the microphone 8 innate to traditional hollow hearing aid shells. A bonding agent 3, such as Factor II silicone bonding enhancer (A-320), adheres the soft bodies of layer 1 and center 2 to the plastic faceplate of the device 4, such as the In'Tech® model 10A faceplate. A typical microphone 8 such as a Knowles® model TM 3546, and a typical acoustic receiver 5 such as a Knowles® model ES7653, are coupled to a typical amplifier 6 such an Etymotic Research model ER-47D K-Amp DSD programmable hybrid, and are populated with capacitors and other components. A typical programming socket 17 such as a Microtronic CS44, is coupled to the amplifier assembly, and serves as the interface between the hearing instrument and the programming unit.

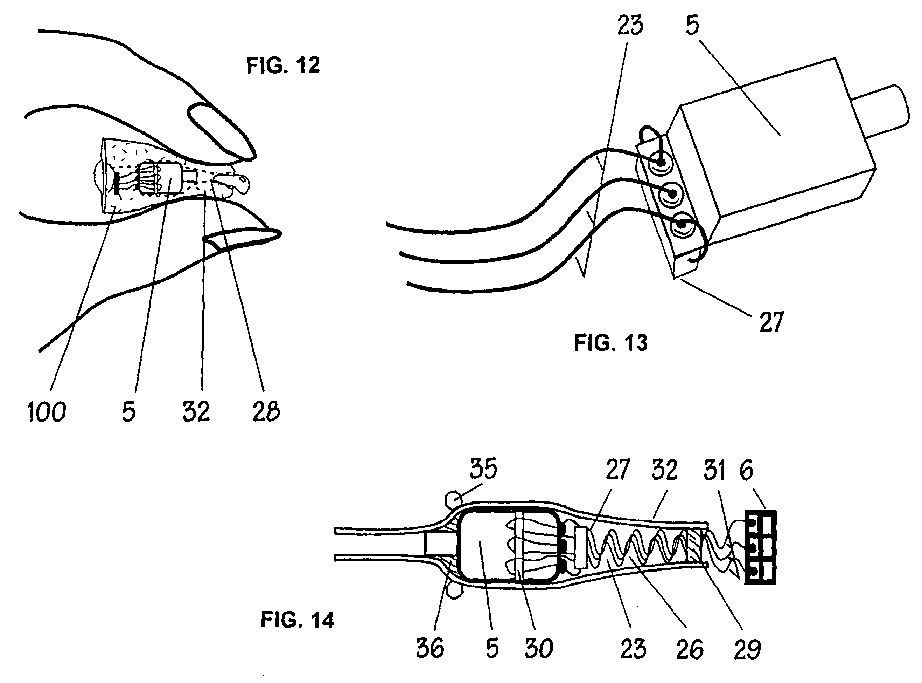

Sound enters the microphone 8 and is transduced into an electrical signal then transmitted by a wire (such as a five strand, forty-four gauge wire) 23 to the amplifier 6. A wiring harness connecting the receiver 5 to the amplifier 6 with an optional but preferred S-shaped loop 26 (see Figure 14) acting as a strain relief allows the two components to move away from each other and back again when the body of the instrument is being squeezed or flexed. When employing high gain and high output, the signal is conducted by wire to a capacitor 27 (see Figure 13) mounted on board the receiver 5, in lieu of the customary location upon the amplifier substrate. This smooths the response curve by reducing the effect of any heterodyning interference due to the antenna effect of the longer receiver wires which are necessary to accommodate the strain relief loop 26. The signal is then transduced back to an acoustic signal by the receiver 5. The acoustic signal then enters a wax guard 15 including a preferably spherical cavity which extends to a preferably cylindrical tube. This cavity is loosely filled with an acoustic medium 16, usually lambs wool or foam, which acts as a wax protection system for the receiver 5, and further serves to smooth out the peaks of the acoustic response. Venting system 14 allows for pressure equalization during swallowing and reduces the low frequency sound spectrum. The vent system 14 can be molded in place using a silicone tube and removing it once the soft-solid hearing aid body has completely vulcanized. In a similar manner, the wax guard 15 can be molded by a tube and sphere which are withdrawn once the body of the device has cured. Both venting system 14 and wax guard 15 are optional depending on the hearing loss and the physical size and shape of the particular ear being fit.

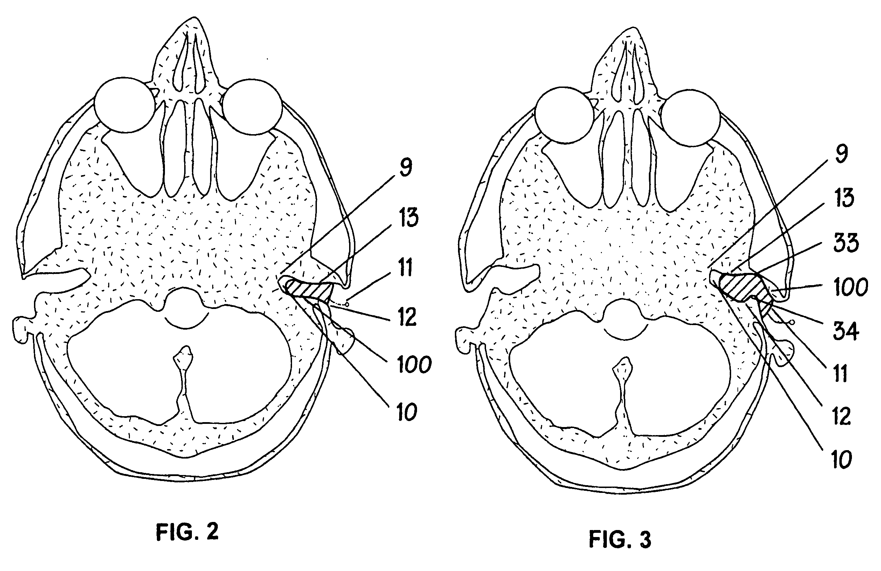

FIG. 2 shows the device 100 in a graphic illustration of a typical human ear as viewed superiorly. The tympanic membrane 9 accepts the output of the soft-solid device 100 There is a snug and uniform acoustic seal between the canal wall 13 and the outer wall 1 of the device 100. This snug and uniform acoustic seal between the canal wall and the outer wall of the device is maintained during jaw motion and head movement.

FIG. 3 shows the device 100 as described in FIG. 2 except with an anatomic variant defined as a bulbous ear canal. The opening to the bulbous external ear canal is geometric narrowed by the anterior 33 and the posterior 34 ear canal surfaces at the first directional bend. The device 100 compresses as it is inserted through the narrowing with the soft solid device layers 1 and 2 returning to their normal state once the device 100 is in its final seated position.

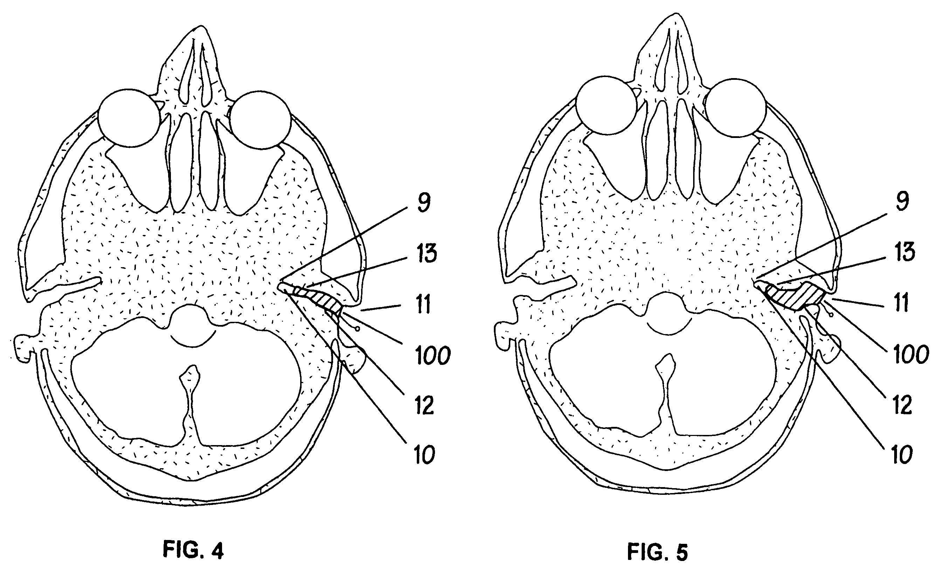

FIG. 4 shows the device 100 as described in FIG. 2 except with an anatomic variant defined as a stenotic ear canal. The ear canal progressively narrows medially. Because of the solid-soft nature of the device, the need to build up the medial tip of the instrument to suspend the receiver is eliminated.

FIG. 5 shows the device as described in FIG. 2 except with an anatomic variant defined as a tortuous ear canal. The ear canal has a sharp turn between the first and second bends, and turns again beyond the second bend. Because of the soft nature of the instrument, the device 100 is able to flex around the canal angles, presenting the desired receiver angle toward the tympanic membrane 9.

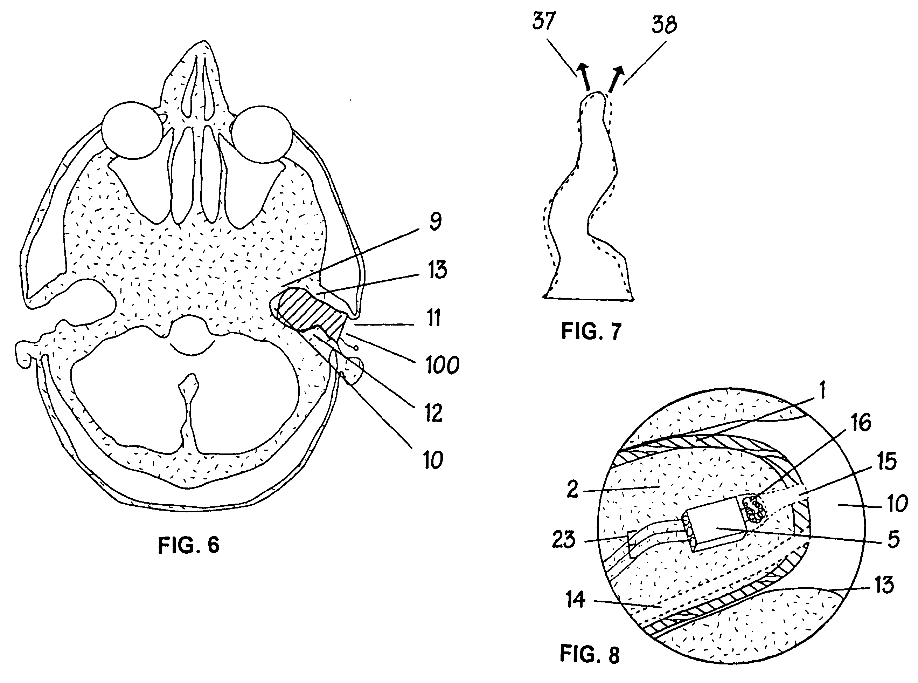

FIG. 6 shows the device as described in FIG. 2 except with an iatrogenic variant demonstrating a post surgical ear canal. The ear canal has been surgically widened, while the rising plane of the ear canal has remained. Because of the firm acoustic seal between the outer skin of the instrument 100 and the external ear canal wall 13, the instrument will resist migration out of the ear canal in response to jaw motion or head movement. Further, the softness of the product reduces the pressure on the facial nerve usually rerouted as part of normal surgical protocol.

FIG. 7 shows the device as described in FIG. 2 demonstrating ear canal volume change and canal angle variance between the jaw-open position 37 and the jaw-closed position 38.

FIG. 8 shows the preferred embodiment of the present invention including the preferred embodiment of the wax chamber 15. Receiver 5 is mounted in the soft-solid 2 inner layer. A cavity is formed by the extreme distal end at the receiver's port medially outward, which is filled with acoustic media 16 to serve as a wax barrier and an acoustic damper which will smooth the acoustic response of the receiver.

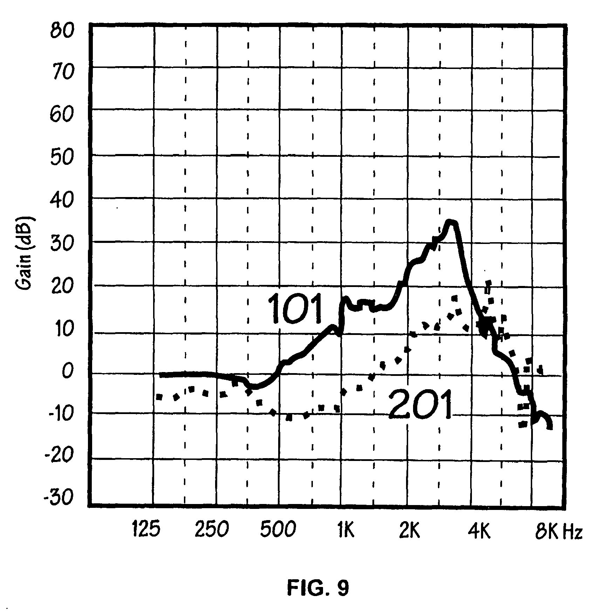

FIG. 9 shows a typical real ear acoustic response of the device 100 before the onset of feedback. Curve 101 represents the soft solid device and curve 201 represents a hard, hollow acrylic device of identical, electro-acoustic design. Curve 101 reveals greater acoustic gain and sound pressure levels before feedback.

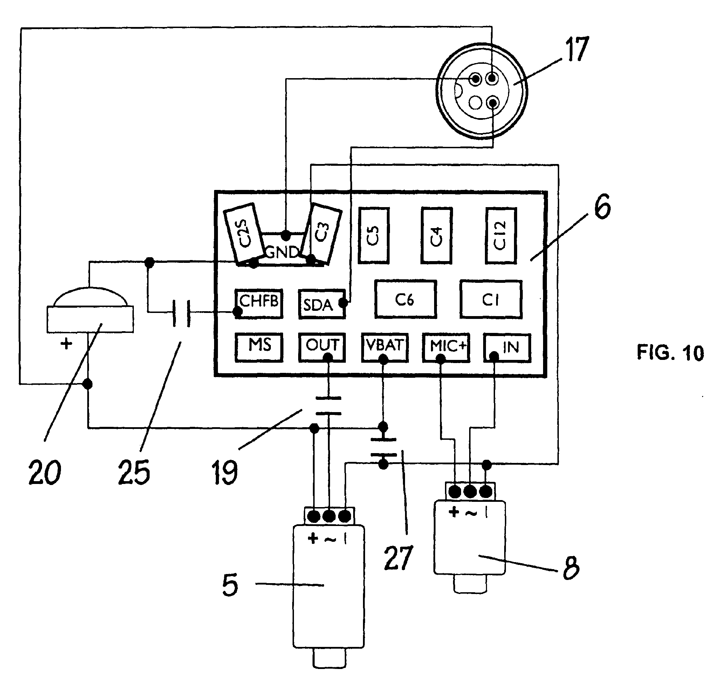

FIG. 10 shows a typical single channel programmable K-Amp 6 hearing aid design being currently produced. Incoming sound enters the microphone 8 where it is transduced to an electrical signal. It then enters the amplifier 6 for processing and amplification, then enters the CHFB capacitor 25 for high frequency signal boost. It then returns to the amplifier and then to the output capacitor 19 which isolates the DC and AC components. The trimming capacitor 27 then filters the signal of distortion caused by heterodyning or other radio frequency interference. The signal is then transduced back into sound in the receiver 5. The programmable adjustments to the microprocessor are addressed through the programming socket 17 and then by the programmer of choice.

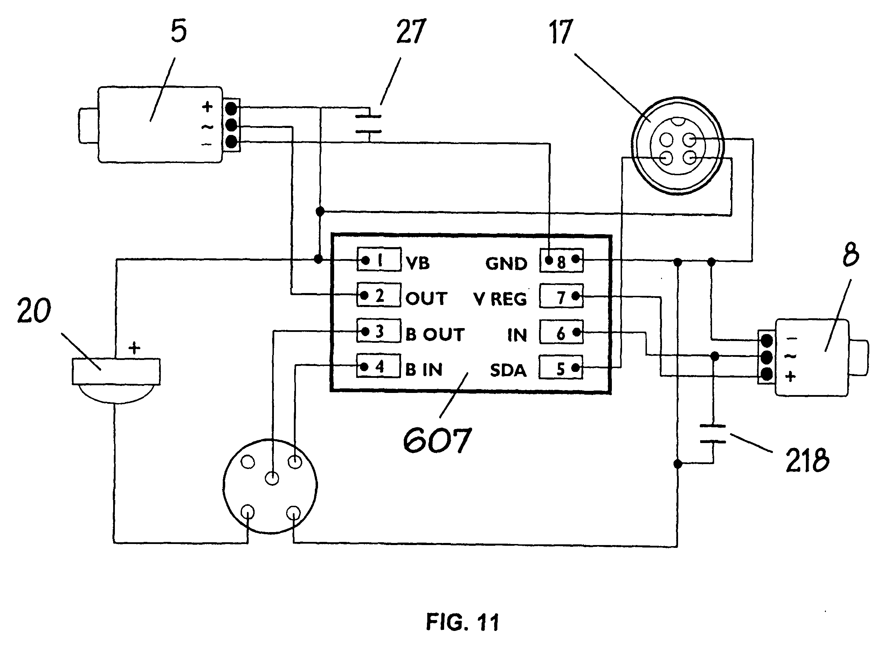

FIG. 11 shows a typical dual channel programmable DEQII hearing design being currently produced. It should be noted that many hearing aid circuits and other types of circuits could be housed in this soft solid embodiment. Examples include 100% digital circuits, such as the Widex Senso, the Oticon Digifocus, the Philips Open-platform digital,the Siemens Prisma, and others to come, and mass communication devices such as: pagers, beepers, cellular phones, digital phones, and composite devices of the above which are currently in development.

FIG. 12 shows an alternative, "tubed wax guard" system 28. The receiver 5 is mounted with silicone tubing 32 of sufficient length (4-8 mm). Visual inspection, since the product is a clear translucent elastomer, reveals the presence of ear wax which is ejected by pinching the device 100 between the index finger and the thumb. Positive pressure is formed by the closed tube to the receiver and relieved to the outside world.

FIG. 13 shows an alternative capacitor location to reduce heterodyning of the amplified signal. The bunching and coiling of the receiver 5 wires in the "S" shaped bundle 26 cause an antenna effect commonly referred to as heterodyning; this is represented as spikes in the acoustic response curve. Mounting the trimming capacitor 27 at the receiver terminal board filters this effect.

FIG. 14 shows the preferred embodiment for the strain relief system 26. The dynamic nature of electronic components embedded in a soft elastomer create the need for hard wiring to be connected to the components and yet maintain the ability to allow the components to move with flexing of the device 100. The receiver 5 is mounted in tubing 32 with the anti-heterodyning capacitor 27 on board the receiver box. The wires 23 are soldered in place and routed to a compression strain relief 30 at the lateral end of the receiver. The wires then enter the tubing and are bunched into an S-shaped bundle 26 forming a compressible and telescopic harness which will accommodate the tensile force, compression force, axial torque, and radial torque exerted by the device 100 across all planes. Plug 29 is used to maintain the air cavity formed between the plug and the receiver lateral end. Receiver 5 is sealed at the extreme distal end at its port 36 to prevent acoustic leakage and cerumen penetration. The wires 23 are then anchored by a tacked strain relief 31 and then connected to the solder pads of the amplifier 6

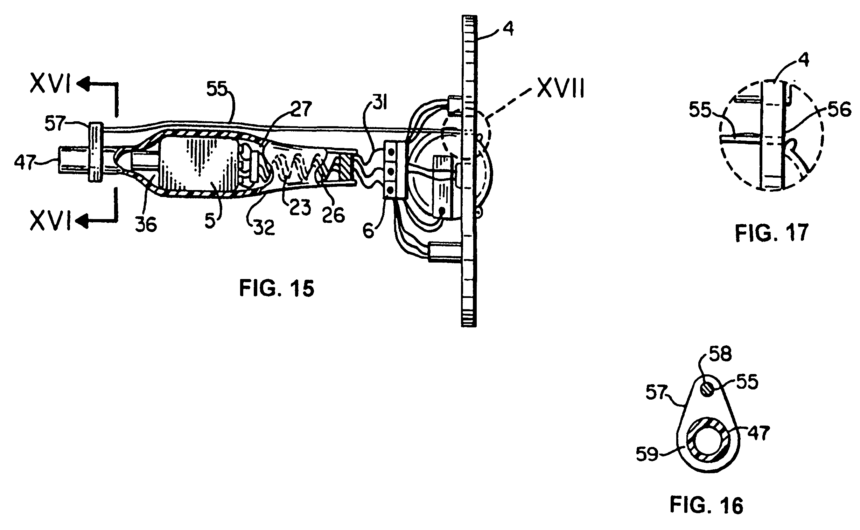

FIGS. 15-17 show a strain relief and receiver positioning system of the preferred embodiment of the present invention. The strain relief and receiver positioning system includes a monofilament cantilever 55 can be used to carry tension so that tension is not transmitted to the wiring harness including wire loops 26. In Figures 15, 16, and 17 the cantilever 55 is anchored to plate 4 at opening 56. A fastener 57 affixes to receiver tube 47 at large opening 59. Monofilament cantilever 55 attaches to fastener 57 at smaller diameter opening 58. The components shown in Figure 15 can be encapsulated with soft solid material as shown in Figure 1.

The monofilament cantilever 55 provides longitudinal stability to the body. It minimizes longitudinal displacement (stretching as well as compression) and thus acts as a longitudinal stabilizer (a longitudinal load carrying member).

Instead of using fastener 57 and monofilament cantilever 55, one could use a double lumen tube to act as receiver tube 47 and as a vent 14 which will also act as strain relief.

Also, instead of using fastener 57 and monofilament cantilever 55, and instead of using a double lumen tube to act as receiver tube 47 and as a vent 14 which will also act as strain relief, one could use a vent tube as vent 14 which is bonded to the soft polymeric body 2 in the same manner as plate 4 is bonded to the soft polymeric body 2; this vent tube remains in place and acts a strain relief means because, being bonded to the soft polymeric body 2, it limits the longitudinal elongation or compression of the soft polymeric body 2.

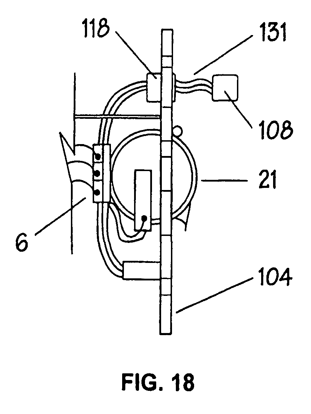

FIG. 18 shows a microphone positioning and assembly system which can be used with traditional hearing aids as well as with the hearing aid of the present invention. This system includes a sleeve 118 mounted in the faceplate 104 into which a microphone 108 can be received. The sleeve 118 could be made of silicone tubing, for example. The sleeve 118 allows the microphone 108 to be removed from the faceplate 104 as shown in Figure 18 to be connected to wires 131 or to be replaced, then microphone 108 is pushed into sleeve 118.

### TYPICAL SOFT-SOLID MATERIALS:

Elastomers: Dow Corning ® MDX4-4210 Base and Dow Corning® MDX4-4210

### Curing Agent

Acetone
Preferred elastomers: Factor II Realistic II Polydimethysiloxane with functional groups and fillers) (A-588, A-588T, or A-588V)
Factor II Silicone Primer (A-304)
Factor II Silicone Bonding Enhancer (A-320)
Audacryl RTC Methacrylate Monomer Mixture
Audacryl RTC Poly Ethyl Methacrylate (clear)

### TYPICAL HEARING AID CIRCUIT COMPONENTS:

Tansitor® 2.2 capacitor
Tansitor® 0.047 capacitor
RTI® 10A hard battery boot
Insulated stranded wire
silicone tubing

### TYPICAL EARMOLD PREPARATION

The following procedure is a description of an alternative, soft-solid body manufactured without the outer skin. The hardness of the soft-solid body is preferably 5-55 Durometer, Shore A, more preferably 5-35 Durometer, Shore A, and most preferably 7-20 Durometer, Shore A.

### A. Preparation of the impression and construction of the acrylic cast

The preferred impression should be of a material such as silicone, since the dimensional stability and elastic memory are crucial to a faithful reproduction of the ear cavity. The use of polymethyl methacrylate is not recommended because of poor dimensional stability (notably, shrinking after 48 hours). The canal length of the impression should extend at least 3 mm past the second bend of the external auditory canal. The open mouth ear impression technique is recommended.

The impression is sized to the desired length, consistent with deep insertion technology. Topographic details of the impression are maintained.

The impression is sprayed with silicone mold release.

A female cast is formed by dipping the impression in uncured acrylic. The cast should have a wall thickness of 2 mm.

The acrylic cast is thermally cured in water or exposed to ultraviolet (UV) light for a two-minute period. The acrylic cast is clear to allow for easy observation of the electronic components during the molding process.

The impression is then removed from the acrylic cast. The lateral face of the acrylic cast is decked down to meet the requirements for the desired hearing instrument style (e.g., CIC, ITE).

### B. Preparation of the electronics

Electronics are assembled on the plate per standard hearing instrument procedure with the following differences:
A full hard battery boot should be used, and should be sealed completely.
Components should not come into contact with the acrylic investment.
The microphone port is drilled into the faceplate, and the microphone is surface mounted. The microphone and looped microphone wiring are sheathed in silicone tubing.

A wiring harness connecting the receiver to the amplifier with an S-shaped loop acts as a dynamic strain relief system. This protects the wire elements and the solder pads on the circuit board and on the receiver during flexion or compression of the instrument.

### C. Preparation of the faceplate

The faceplate is prepared with a coating of acetone, and is set aside to dry.

Once the acetone coating has dried, the primer is applied and the faceplate is set aside to dry for a thirty minute period.

Following that, a coating of the bonding enhancer is applied and is set aside for a thirty minute drying period.

### D. Closing procedure

The receiver port, the vent port, and a filling port are drilled into the acrylic cast. The inside of the acrylic cast is thoroughly cleared of debris.

The receiver tube and the vent tube are positioned in the cast.

The electronics are positioned into the cast. Thee faceplate is mounted onto the cast, and is cycobonded to the cast.

### E. Filling procedure

The soft elastomer material (preferably Factor II Realistic II Polydimethysiloxane with functional groups and fillers, A-588, A-588T, or A-588V) is injected through the filling port via syringe. Any existing air bubbles should be eliminated (e.g., by pulling a vacuum or adding a thixotropic agent such as Thixo available from Factor II).

The microphone and receiver tubes are sealed with elastomer plugs to prevent water from infiltrating the transducers.

The elastomer in the filled shell is thermally cured in water maintained at 120 degrees Fahrenheit (water is sometime preferred because of the better heat transfer characteristics of water, though air could be used as there is less chance of damaging the electronic components if air is used to thermally cure the elastomer). The temperature may be increased to quicken the curing time, but should preferably not exceed 140 degrees Fahrenheit.

### F. Finishing procedure

The acrylic cast is scored with a metal bit. Snips are used to clip along the scored areas and to remove the cast from the instrument.

Once the cast has been removed, a heated ruby bit is used to finish the seam between the body of the body and the faceplate of the instrument.

The soft-solid hearing instrument of the present invention has a soft-solid nature which provides an environment for the electronic circuit that will improve transducer performance, improve transducer longevity, and will be more resistant to vibration, force, and shock, as compared to hollow hearing aids. Because of its soft nature, it will provide greater comfort, will resist migration out of the ear canal, and will remain acoustically sealed with jaw excursion. Because of its soft nature, it will allow for the full utilization of advanced electronic performance of which high fidelity programmable and digital designs are capable.

Because of its soft nature and because its superior acoustic seal, it reduces or eliminates unwanted acoustic feedback. Because of its soft nature, it will be easily insertable, will accommodate anatomical aberrations, and will--because of its capability of being deeply inserted beyond the second anatomical bend--provide increased perceived power. Because of its solid nature, it will have no internal surfaces, thereby eliminating internal reflection and reverberation, eliminating the internal feedback path between the receiver and the microphone.

Coloring of the elastomer is possible using two methods. The first method is intrinsic, under the surface layer. The second method is extrinsic, on the surface layer. The methods can be done separately or in conjunction with one another.

The preferred method is intrinsic coloring, whereby a special dye is mixed thoroughly into the uncured material in very small ratios (e.g., 1 drop per 10 grams). Once the mixture is completed, the material is cured in the same way as would be the uncolored material. An alternative method is extrinsic coloring, whereby an extrinsic tri fluid solvent is applied to the surface of the cured material, causing the pores of the material to open. At that point, dye is brushed onto the surface of the material. As the solvent dries, the pores of the material close, and the dye is enveloped.

Service of the electronic components is accomplished by making an incision in outer layer 1 and center layer 2 until the damaged component is exposed for repair or replacement. Material bonding approach (also addresses repair issue): the preferred method involves cleaning the damaged site on the instrument with alcohol and allowing it to dry. A thin coat of bonding enhancer (A-320) is applied to the site, which is then allowed to dry for thirty minutes. Once the bond enhancer has dried, a layer of soft-solid material is applied. The instrument is then cured for thirty minutes in an oven which has been preheated to 60 degrees C. If necessary, the repaired site can be sculpted to conform to the original shape of the instrument.

An alternative method involves applying a layer of acetic acid based elastomer onto the damaged site on the instrument. That elastomer serves to open the pores of the material, bonding the materials. The instrument is then cured for thirty minutes in an oven which has been preheated to 60 degrees C.

Some preferred assembly and repair procedures follow:

### 1. SoftEar UVC3 casting procedure

To build this product correctly, we must insist that a thorough and complete impression with only minimal voids from otoblock lines be made. Those voids should be filled with wax, modeling clay, or another medium which will not add to the diameter of the canal. The length of the canal should, at the very least, extend beyond the second bend with the sound bore directed at the tympanic membrane.
1. The impression is cut to length as dictated by the desired style. Very little preparation should be done to the impression, and only then to slightly round off sharp edges that may cause discomfort to the wearer.
2. As a standard, the impression is dipped three times in pink wax kept at 200 °F (we have selected extra tough pink, dental base-plate wax manufactured by Hygenic).
3. That impression is invested in clear two-part silicone manufactured by Dreve. That two-part silicone is dispensed after having been blended in a Dreve dosper. The investment is typically cured at room temperature for approximately 10 minutes, but may be cured in a heated pressure pot at approximately 251b of pressure at 115°F for approximately 7 minutes. That step will ensure a minimal amount of air bubbles in the investment.
4. UV material is poured into the investment (we are currently using Dreve as a UV chemical supplier), the material is cured for approximately 1.5 minutes (we are currently using a UVA Polylux 8 curing chamber manufactured by Dreve).
5. Following the first curing stage, excess UV material is recovered. The shell cavity is then filled with glysol solution, and cured in the UVA chamber for 4 minutes. The shell is removed from the silicone investment.
6. The outside of the cast can be built up for structural stability, and is transferred to the electronics lab.

### 2. SoftEar UV-Exact casting procedure

This is procedure will result in absolute fidelity to the ear canal impression. All features of the impression will be in evidence in the final product.

To build this product correctly, we must insist that a thorough and complete impression with only minimal voids from otoblock lines be made. Those voids should be filled with wax, modeling clay, or another medium which will not add to the diameter of the canal. The length of the canal should, at the very least, extend beyond the second bend with the sound bore directed at the tympanic membrane.
1. The impression is cut to length as dictated by the desired style.
2. The impression is sprayed with a mold-release agent (we have selected Ease Release 200 manufactured by Mann Formulated Products).
3. The sprayed impression is dipped in photoplast gel (we have selected Keystone Gel # TG-71. Fotoplast gel from Dreve can be substituted if need be.)
4. The coated impression is placed in the UVA curing chamber and is cured for approximately 3.5 minutes.
5. The impression is removed from the cast
6. The cast is cleared of any debris, is placed in a glysol bath, and cured in the UVA chamber for 4 minutes.
7. The cast is removed from the UVA chamber. Holes are then drilled for the receiver tube and for injection of the material. Keep in mind that the "injecting holes" should be drilled in areas which allow for filling of the cast without the trapping of air bubbles. The cast is then passed to electronics for loading of the components.

### 3. SoftEar preferred method of assembly and stabilization:

While any type of circuit can be used with the SoftEar procedure, our preferred procedure involves digitally programmable circuitry.
1. Into the top eye of the stabilizer fitting, an length of 30# test monofilament, which serves as the cantilever arm, is installed.
2. Onto the desired receiver, receiver wires are attached to the pads. A full boot is installed, leaving approximately 2 mm extended over the solder pads and leads.
3. Into the 2 mm extension, wire is folded over and tamped in to form an "s loop" which serves as a strain relief system. The end of the extension is then sealed with a silicone plug. Along the outside of the receiver boot, a small bead of silicone is installed on each side of the receiver which facilitates centering the receiver in the cast during the filling procedure.
4. Onto the port of the receiver, receiver tubing is glued. The receiver tubing is of a diameter to fit through the bottom eye of the stabilizer fitting.
5. The receiver wires, the microphone wires (to which the microphone is not yet attached), and - if needed- the volume control wires are attached to the hybrid.
6. Onto a standard faceplate, hard terminal wires are installed.
7. A hard boot is installed over the battery contacts. If the boot has open sides, tape is applied to the outside of the boot, and fixed with cyanoacrylate.
8. On the faceplate the holes for the microphone and the programming socket are drilled, and a programming socket is installed into the faceplate.
9. A jig with the dimensions of the hybrid to be used is employed to determine the optimum circuit location (determined by the cast supplied by the shell lab).
10. The jig is replaced with the hybrid, which is attached to the desired location. To provide extra strain relief, the receiver wires are routed below the hybrid contacts and are secured to either the hybrid or the battery boot. The programming socket is wired to the hybrid.
11. Into the microphone hole, a grommet is installed and the microphone wires are pulled through the grommet. The grommet is then sealed with silicone.
12. The hole for the cantilever assembly is then drilled into the faceplate.
13. The receiver tube is pulled through the bottom eye of the stabilizer fitting.
14. The cantilever arm is pulled through the hole in the faceplate. The cantilever arm is not secured at this point.
15. The assembly is then inserted into the cast. The cantilever arm is manipulated until the receiver is at the desired site in the cast. The receiver is not secured at this time. The cantilever arm extending from the lateral side of the faceplate is clamped. The cast is removed from the assembly. The cantilever arm is cut flush with the outside of the faceplate. The arm is pushed through to the medial side of the faceplate, the tip of the arm is melted, and cyanoacrylate is applied to that area. The arm is reinserted into the faceplate so that it is flush with the lateral side of the faceplate.
16. The vent hole is drilled into the faceplate.
17. The assembly is then returned to the shell lab to be prepped for mounting.

### 4A. SoftEar faceplate preparation for closing

1. Acetone is painted onto the faceplate, within the candled areas on the medial side. Care is taken to not directly apply acetone to the wires or the components. This is allowed to dry for about 3 minutes.
2. The Primer is then added to the area that was previously covered with acetone. Due to the importance of this material in the bonding process, the Primer must be allowed to dry for 30 minutes.
3. Bonding Enhancer is then applied ,and allowed to dry for approximately 20 minutes.

### 4B.SoftEar closing procedure:(1)

1. The vent tubing is placed inside the cast.
2. The electronics are inserted into the cast, the receiver tube is pulled through the receiver tip, and the vent tube is pulled through the faceplate. The extended receiver tube is plugged with silicone to prevent contamination during the filling and curing process.
3. The prepared plates are then married to the cast by using a mix of cyanoacrylate and polymer. Ensure that the polymer bond is thick enough to prevent seepage under the seam and compromise of the bond at the outer edge of the product.
4. The unit is then prepared for filling.
5. SoftEar filling procedure

1. The elastomer is blended in a 1:1 ratio. As a rule, about 7 grams of material are required for each instrument. If the mixture is too thick, functional fluid can be added to thin the blend, but the functional fluid should not exceed more than 10% of the total mixture..
2. The blended elastomer is subjected to 20 pounds of pressure for 2 minutes. The mix is then placed in an evacuator for five minutes. The mixture is again placed under 20 pounds of pressure for 2 more minutes. The result should be a bubble-free material.
3. The material is drawn into a syringe for injection into the cast.
4. When filling the cast, best results are obtained by filling from the injection port near the plate, allowing the material to flow to the tip of the canal. Any bubbles trapped during the process can be forced out through the other ports.
5. Once the cast is filled, a final check of the receiver and microphone ports is done to ensure they are completely water tight. The filled instrument is placed in a water bath and kept at 110°F for 1 hour. Care should be taken to prevent the temperature from exceeding 140°F to avoid damaging the electronic components.
6. SoftEar final finish procedure

### Removing the instrument from the cast

1. The cured instrument is removed from the water bath. The cast is scored along its length with a metal burr.
2. Snips are used to cut along the score lines, and the pieces of the cast are removed from the elastomer body.
3. The faceplate is finished in a traditional manner.
4. The instrument is then ready for mounting the microphone.

### Mounting the microphone

I. The grommet and the silicone plug are removed from the mic port. The mic wires are cut to the point that they extend 4 mm from the faceplate.
2. A windscreen is installed onto the mic. The mic wires are soldered onto the pads. The pads are insulated with a piece of Ad-Hear that has been cut to fit.
3. The mic is mounted into the port in the faceplate. The mic is stabilized in place by sealing the windscreen to the faceplate with monomer.

### Pre-QA preparation of the instrument.

1. The receiver tube and the real ear tube are cut to the point that they extend 2 mm from the receiver tip.
2. Following ANSI standards, the instrument is assessed to rule out internal feedback or other electroacoustic anomalies. If anomalies are present, the instrument is returned to the technician.
3. If the circuit used is a programmable unit, the instrument is interfaced with the programming instrumentation. The instrument is programmed to achieve desired parameters (we have chosen Fig6).
4. The finish of the instrument is assessed. If there are elastomer nodules or any other faults, the instrument is returned to the shell lab.
5. If the instrument meets pre-QA criteria, the instrument is sent to QA.
7. SoftEar Recommended QA procedure

### Electronics:

1. Ensure that the Production Information slip accompanies the order in duplicate. If not, return it to Shell Lab.
2. Do not cut any tubing extending out of the faceplate. This is especially true of the real ear tubing whether it is fed through the vent tubing or is used as the vent tubing itself. At the receiver tip, let the real ear tube extend by 2mm whenever possible.
3. Ensure that the instrument has a serial number which agrees with the picking ticket. If it does not, return it to Shell Lab.
4. If a programmable circuit has been used, ensure that the instrument has been programmed by an audiologist. If not, return the instrument to Electronics Lab.
5. Ensure that the investment and the impression is with the instrument. If not, return it to Shell Lab.
6. As with normal QA procedure, if any electronic anomaly is present, return the instrument to Electronics Lab.
7. If the instrument meets Electronics QA criteria, pass it on to Final QA. SoftEar recommended QA procedure

### Final QA:

1. Examine the instrument carefully for imperfections. Any detection of holes, separation at the faceplate, or cuts in the finish should result in returning the instrument to Shell Lab.
2. Examine the receiver tip. IfTygon tubing has been used, clip the receiver tubing so that it is recessed 1 mm from the receiver tip surface. If doing so will result in contacting the receiver port itself, consult with the Production Manager.
3. Whenever possible, let the real ear tubing extend by 2mm.
4. Ensure that the receiver tip is smooth, and free of any elastomer nodules. If not, return the instrument to Shell Lab.
5. Ensure that the serial number, the investment, and the impression are present. In addition, ensure that the Product Information sheet is present in duplicate. If not, return it to Shell Lab.
6. During the listening check, follow normal QA procedures. If any failure is detected, return it to Electronics Lab.
7. If the instrument meets Final QA criteria, pass it on to Shipping.

### SoftEar recommended Shipping procedure

### Shipping:

1. Ensure that all paper work is in order. Make certain that one copy of the Product Information sheet accompanies the GHI file copy, and one copy accompanies the paperwork going to the customer.
2. Ensure that the investment and the impression are with the instrument. Return the investment and the impression to the designated SoftEar storage area.
3. Follow normal Shipping procedures.
8. SoftEar electronic repair

### Microphone repair

4. If the FG-3329 mic has been used, the windscreen is removed and the mic is pulled free of the faceplate, and the wires are separated from the mic.
5. A new mic/windscreen assembly is attached to the wires, and the pads are insulated.
6. The new mic is inserted into the mic port, and the windscreen is sealed to the faceplate with monomer.

### Receiver repair

1. A small cut is made into the elastomer perpendicular to the length of the receiver at the site of the receiver pads.
2. The receiver is removed from the body of the instrument through that cut, and the wires are separated from the receiver. It should be noted that the receiver tubing will stay in place.
3. The new receiver wires are attached to the receiver. A tubing spreader is used to distend the small cut from which the old receiver was removed.
4. The port end of the receiver is inserted into the distended cut, and is guided into the receiver tubing.
5. Once the pad end of the receiver has been returned to the desired sit, the instrument is sent to the Shell Lab for sealing.
9. SoftEar body repair

### Repair body following electronic repair

1. The slit is treated with primer, which is allowed to dry for 3 minutes.
2. The slit is then treated with bonding enhancer, which is allowed to dry for 3 minutes.
3. The slit is filled with elastomer blend, ensuring that no bubbles infiltrate the mixture. Any excess material can be stored in the syringe under refrigeration for several days.
4. The instrument is placed in a convection oven for 30 minutes at 60°C (140°F).
5. Any excess elastomer can be removed using a ruby studded bit.

### Modification of body for better fit

1. A new cast is formed from a fresh impression using the casting technique desired. The tip of the new cast is removed to serve as the primary filling hole. An over-fill hole should be drilled at the area of the aperture.
2. The elastomer is pared away from the receiver tip toward, but not including, the faceplate. Care should be taken to ensure that all internal components remain embedded in elastomer.
3. The trimmed instrument surface is coated with primer which is allowed to dry for 3 minutes.
4. The primed instrument is then placed into the new cast. Care is taken to ensure correct alignment of components, and good conformity to the faceplate.
5. A syringe is filled with freshly blended elastomer. The elastomer is injected from the receiver end of the cast. Care should be taken to ensure the cast is completely filled, and that the elastomer is free of bubbles. Elastomer should exude through the over-fill hole.
6. The recast instrument is placed in a convection oven set at 60°C (140°F), and allowed to cure for 1 hour.
7. The cast is removed in the same manner as with a new instrument, and normal SoftEar finishing techniques are employed.
The following table lists the parts numbers and parts descriptions as used herein and in the drawings attached hereto.

### PARTS LIST:

The following is a list of parts and materials suitable for use in the present invention:
- 1: soft-solid outer skin (optional)
- 2: soft-solid inner body
- 3: bonding agent
- 4: faceplate (such as an In' Tech® 10A Faceplate)
- 5: receiver (such as a Knowles® ES7653 receiver)
- 6: amplifier (such as an Etymotic Research ER-42D K-Amp®/DSD programmable hybrid)
- 7: vent hole in faceplate 4
- 8: microphone (such as a Knowles® TM3546 microphone)
- 9: TM
- 10: cavity
- 11: concha
- 12: sagittal plain or aperture of ear canal
- 13: external ear canal wall
- 14: vent in hearing aid
- 15: wax guard
- 16: acoustic media (such as lambs wool or foam) in wax guard
- 17: programming socket (such as a Microtronic CS44 socket)
- 18: input decoupling capacitor
- 19: output decoupling capacitor
- 20: battery
- 21: battery door (such as an In'Tech® 10A Battery Door)
- 22: extraction cord
- 23: 44 gauge 5 strand wire
- 24: volume control
- 25: CHFB capacitor
- 26: "S" loop strain relief
- 27: Trimming cap for heterodyning
- 28: alternate wax guard system
- 29: Adhesive plug
- 30: Compression strain wire relief
- 31: Tacked strain wire relief
- 32: standard silicone receiver tubing.
- 33: Anterior external ear canal wall
- 34: Posterior external ear canal wall
- 35: Centering nubbins
- 36: Receiver silicone seal
- 37: Open-jaw position
- 38: Closed-jaw position
- 47: receiver tube
- 55: monofilament cantilever
- 56: opening in plate 4
- 57: fastener
- 58: smaller diameter opening in fastener 57
- 59: larger diameter opening in fastener 57
- 100: Soft-solid hearing aid of the preferred embodiment of the present invention
- 104: faceplate
- 108: microphone
- 118: sleeve
- 131: wires

The body could be homogeneous (the outer layer 1 could be left out). Any suitable type of electronic schematic could be used instead of the schematic shown in Fig. 11.

All measurements disclosed herein are at standard temperature and pressure, at sea level on Earth, unless indicated otherwise. All materials used or intended to be used in a human being are bio-compatible, unless indicated otherwise.

The foregoing embodiments are presented by way of example only; the scope of the present invention is to be limited only by the following claims.

## Claims

1. An ear-worn hearing device (100), the device comprising:
(a) a hearing device body sized and shaped to generally fit into a human ear canal; and
(b) an electronic hearing circuit embedded in the body,
**characterised in that** the body is non-hollow, is a soft-solid made of an elastomer and has a Durometer Hardness, Shore A, of less than 40 points.

2. A device as claimed in claim 1, wherein the body includes an outer skin portion (1) and an inner filler portion (2), and at least 90% of the body has a Durometer Hardness, Shore A, of less than 25 points and comprises the inner filler portion.

3. A device as claimed in claim 1, wherein the body includes an outer skin portion (1) and an inner filler portion (2), and at least 80% of the body has a Durometer Hardness, Shore A, of less than 35 points and comprises the inner filler portion.

4. A device as claimed in claim 1, wherein the body includes an outer skin portion (1) and an inner filler portion (2), and the inner filler portion is gel elastomer filled and has a Durometer Hardness, Shore A, of less than 35 points.

5. A device as claimed in any one of claims 1 to 4, wherein the body occupies at least 70% of the volume of the hearing device not occupied by the electronic hearing circuit.

6. A device as claimed in any one of claims 1 to 4, wherein the body occupies at least 80% of the volume of the hearing device not occupied by the electronic hearing circuit.

7. A device as claimed in any one of claims 1 to 4, wherein the body occupies at least 90% of the volume of the hearing device not occupied by the electronic hearing circuit.

8. A device as claimed in any one of claims 1 to 4, wherein the body occupies at least 99% of the volume of the hearing device not occupied by the electronic hearing circuit.

9. A device as claimed in any preceding claim, wherein the body is non-absorbent and substantially impervious to cerumen.

10. A device as claimed in any preceding claim, wherein the body shields, by means of encapsulation, the electronic hearing circuit from the hostile environment of the ear which usually causes corrosion of exposed connections.

11. A device as claimed in any preceding claim, wherein the device is a hearing aid having a receiver (5), a receiver port, and a medial end, and the receiver (5) is recessed from the medial end of the instrument, thereby allowing cerumen to be extruded from the receiver port when pressure is exerted between the receiver and the medial end of the device.

12. A device as claimed in any preceding claim, wherein the body comprises a blend of elastomer and conductive particles to provide a static shield protecting the circuitry from RFI, GSM and EMI.

13. A device as claimed in any preceding claim, wherein the electronic hearing circuit includes a receiver (5), an amplifier (6), and a wiring harness connecting the receiver (5) to the amplifier (6) with an S-shaped loop (26).

14. A device as claimed in any preceding claim, wherein the electronic hearing circuit includes transducers and the body, because of its soft-solid nature, provides shock absorption for the transducers.

15. A device as claimed in any preceding claim, cast from a standardized impression of the ear, or from several selected sizes of the ear, so as to provide a platform to manufacture a series of non-custom, soft-solid devices.

16. A device as claimed in any preceding claim, wherein the body has a sufficiently low Durometer Hardness Shore A, value so as to allow the device to be worn by the user while sleeping.

17. A device as claimed in any preceding claim, wherein the electronic hearing circuit includes hearing aid circuitry.

18. A device as claimed in any preceding claim, wherein the device is a completely-in-the-canal device.

19. A device as claimed in any preceding claim, wherein the body is custom molded.

20. A device as claimed in claim 1, wherein at least 90% of the body has a Durometer Hardness, Shore A, of less than 25 points.

21. A device as claimed in claim 1, wherein at least 80% of the body has a Durometer Hardness, Shore A, of less than 35 points.

22. A device as claimed in claim 1, wherein the body is gel elastomer and has a Durometer Hardness, Shore A, of less than 35 points.

23. A device as claimed in claim 1, wherein the body comprises a gel and has a Durometer Hardness, Shore A, of less than 35 points.

24. A device according to claim 1, wherein the device (100) is a completely in-the-canal hearing device, and whereby
(a) the hearing device body is custom molded; and
(b) the electronic hearing circuit comprises an amplifier network including an electronic hearing aid circuit, transducers, and volume control, the amplifier network being embedded in the body.

25. A device according to claim 1, wherein the device (100) is a hearing device that is worn inside a human ear canal, and whereby
(a) the body includes an outer surface material and a filler material;
(b) the electronic hearing circuit comprises an amplifier network contained within the filler material, the amplifier network comprising an electronic hearing aid circuit, transducers, and volume control, wherein:
the outer surface material and the filler material have different hardness values, at least one having a Durometer Hardness, Shore A, value in the range of 1 to 7 points, and
the filler material encapsulates the amplifier network to eliminate air spaces that could otherwise cause an internal air conduction path between a receiver (5) and a microphone (8) in the amplifier network.

## Patentansprüche

1. Im Ohr tragbare Hörvorrichtung (100), wobei die Vorrichtung, aufweist:
(a) einen Hörvorrichtungskörper der dimensioniert und geformt ist, um allgemein in einen menschlichen Gehörgang zu passen; und
(b) einer elektronischen Hörschaltung, die in den Körper eingebettet ist,
**dadurch gekennzeichnet, dass** der Körper nicht hohl ist, ein Weichkörper ist, der aus einem Elastomer hergestellt ist, und eine Durometer-Härte von weniger als 40 Punkten Shore A aufweist.

2. Vorrichtung nach Anspruch 1, wobei der Körper ein äußeres Hautteil (1) und ein inneres Füllteil (2) aufweist, und wobei zumindest 90 % des Körpers eine Durometer-Härte von weniger als 25 Punkten Shore A aufweist und das innere Füllteil aufweist.

3. Vorrichtung nach Anspruch 1, wobei der Körper ein äußeres Hautteil (1) und ein inneres Füllteil (2) aufweist, und wobei zumindest 80 % des Körpers eine Durometer-Härte von weniger als 35 Punkten Shore A aufweist und den inneren Füllteil aufweist.

4. Vorrichtung nach Anspruch 1, wobei der Körper ein äußeres Hautteil (1) und ein inneres Füllteil (2) aufweist, und wobei der innere Füllteil ein gefülltes Gelelastomer ist und eine Durometer-Härte von weniger als 35 Punkten Shore A aufweist.

5. Vorrichtung nach einem der Ansprüche 1 bis 4, wobei der Körper zumindest 70 % des Volumens der Hörvorrichtung einnimmt, welches nicht von der elektronischen Hörschaltung eingenommen wird.

6. Vorrichtung nach einem der Ansprüche 1 bis 4, wobei der Körper zumindest 80 % des Volumens der Hörvorrichtung einnimmt, welches nicht von der elektronischen Hörschaltung eingenommen wird.

7. Vorrichtung nach einem der Ansprüche 1 bis 4, wobei der Körper mindestens 90 % des Volumens der Hörvorrichtung einnimmt, welches nicht von der elektronischen Hörschaltung eingenommen wird.

8. Vorrichtung nach einem der Ansprüche 1 bis 4, wobei der Körper mindestens 99 % des Volumens der Hörvorrichtung einnimmt, welches nicht von der elektronischen Hörschaltung eingenommen wird.

9. Vorrichtung nach einem der vorhergehenden Ansprüche, wobei der Körper nicht absorbierend und für Ohrenschmalz undurchlässig ist.

10. Vorrichtung nach einem der vorhergehenden Ansprüche, wobei der Körper die elektronische Hörschaltung mittels Verkapselung gegen die feindliche Umgebung des Ohrs abschirmt, die üblicherweise eine Korrosion freiliegender Verbindungen hervorruft.

11. Vorrichtung nach einem der vorhergehenden Ansprüche, wobei die Vorrichtung eine Hörhilfe mit einem Empfänger (5), einem Empfängeranschluss und einem medialen Ende ist, und wobei der Empfänger (5) von dem medialen Ende des Instruments vertieft ist, wodurch Ohrenschmalz aus dem Empfängeranschluss herausgepresst werden kann, wenn ein Druck zwischen dem Empfänger und dem medialen Ende der Vorrichtung ausgeübt wird.

12. Vorrichtung nach einem der vorhergehenden Ansprüche, wobei der Körper eine Mischung aus einem Elastomer und leitenden Partikeln aufweist, um eine statische Abschirmung zu ermöglichen, die die Schaltung gegenüber HF-Störungen, GSM und elektromagnetischen Störungen schützt.

13. Vorrichtung nach einem der vorhergehenden Ansprüche, wobei die elektronische Hörschaltung einen Empfänger (5), einen Verstärker (6) und einen Kabelbaum aufweist, der den Empfänger (5) mit dem Verstärker (6) mit einer S-förmigen Schlaufe (26) verbindet.

14. Vorrichtung nach einem der vorhergehenden Ansprüche, wobei die elektronische Hörschaltung Umformeinrichtungen umfasst, und wobei der Körper aufgrund seiner Weichkörpernatur eine Stoßabsorption für die Umformeinrichtungen ermöglicht.

15. Vorrichtung nach einem der vorhergehenden Ansprüche, die aus einem standardisierten Abdruck des Ohrs oder aus verschiedenen, ausgewählten Größen des Ohrs gegossen ist, um so eine Plattform zum Herstellen einer Reihe von nicht individuell hergerichteten Weichkörpervorrichtungen vorzusehen.

16. Vorrichtung nach einem der vorhergehenden Ansprüche, wobei der Körper eine ausreichend geringe Durometer-Härtewert Shore A aufweist, um es so zu ermöglichen, dass die Vorrichtung von dem Benutzer während des Schlafens getragen werden kann.

17. Vorrichtung nach einem der vorhergehenden Ansprüche, wobei die elektronische Hörschaltung eine Hörhilfeschaltung umfasst.

18. Vorrichtung nach einem der vorhergehenden Ansprüche, wobei die Vorrichtung vollständig in den Gehörgang einsetzbar ist.

19. Vorrichtung nach einem der vorhergehenden Ansprüche, wobei der Körper kundenspezifisch gegossen ist.

20. Vorrichtung nach Anspruch 1, wobei mindestens 90 % des Körpers eine Durometer-Härte von weniger als 25 Punkten Shore A aufweist.

21. Vorrichtung nach Anspruch 1, wobei mindestens 80 % des Körpers eine Durometer-Härte von weniger als 35 Punkten Shore A aufweist.

22. Vorrichtung nach Anspruch 1, wobei der Körper ein Gelelastomer ist und eine Durometer-Härte von weniger als 35 Punkten Shore A aufweist.

23. Vorrichtung nach Anspruch 1, wobei der Körper ein Gel aufweist und eine Durometer-Härte von weniger als 35 Punkten Shore A aufweist.

24. Vorrichtung nach Anspruch 1, wobei die Vorrichtung (100) vollständig in den Gehörgang einsetzbar ist, und wobei
(a) der Hörvorrichtungskörper kundenspezifisch gegossen ist; und
(b) die elektronische Hörschaltung ein Verstärkernetzwerk aufweist, einschließlich einer elektronischen Hörhilfeschaltung, Umformeinrichtungen und einer Lautstärkenregelung, wobei das Verstärkernetzwerk in den Körper eingebettet ist.

25. Vorrichtung nach Anspruch 1, wobei die Vorrichtung (100) eine Hörvorrichtung ist, die innerhalb eines menschlichen Gehörgangs getragen wird, und wobei
(a) der Körper ein äußeres Oberflächenmaterial und ein Füllmaterial umfasst;
(b) die elektronische Hörschaltung ein Verstärkernetzwerk aufweist, das in dem Füllmaterial enthalten ist, wobei das Verstärkernetzwerk eine elektronische Hörhilfeschaltung, Umformeinrichtungen und eine Lautstärkenregelung aufweist, wobei:
das äußere Oberflächenmaterial und das Füllmaterial unterschiedliche Härtewerte aufweisen, wobei zumindest eines einen Durometer-Härtewert in dem Bereich von 1 bis 7 Punkten Shore A aufweist, und
das Füllmaterial das Verstärkernetzwerk einkapselt, um Lufträume zu eliminieren, die ansonsten einen inneren Luftleitungsweg zwischen einem Empfänger (5) und einem Mikrofon (8) in dem Verstärkernetzwerk hervorrufen könnten.

## Revendications

1. Dispositif auditif à porter sur l'oreille (100), comprenant :
(a) un corps de dispositif auditif de taille et de forme adaptées de manière générale au canal auditif humain ; et
(b) un circuit auditif électronique incorporé dans le corps,
**caractérisé en ce que** le corps n'est pas creux, et est réalisé en un matériau souple / solide d'un élastomère et présente une dureté Duromètre, Shore A, de moins de 40 points.

2. Dispositif selon la revendication 1, **caractérisé en ce que** le corps inclue une partie (1) de peau extérieure et présente une partie (2) de remplissage intérieur, et au moins 90% du corps présente une dureté Duromètre, Shore A, de moins de 25 points et comporte la partie de remplissage intérieur.

3. Dispositif selon la revendication 1, **caractérisé en ce que** le corps inclue une partie (1) de peau extérieure et une partie (2) de remplissage intérieur, et au moins 80 % du corps présente une dureté Duromètre, Shore A, de moins de 35 points et comporte la partie de remplissage intérieur.

4. Dispositif selon la revendication 1, **caractérisé en ce que** le corps inclue une partie (1) de peau extérieure et une partie (2) de remplissage intérieur, et la partie de remplissage intérieur est remplie de gel élastomère et présente une dureté Duromètre, Shore A, de moins de 35 points.

5. Dispositif selon l'une des revendications 1 à 4, **caractérisé en ce que** le corps occupe au moins 70% du volume du dispositif auditif, qui est occupée par le circuit d'audition électronique.

6. Dispositif selon l'une des revendications 1 à 4, **caractérisé en ce que** le corps occupe au moins 80% du volume du dispositif auditif qui n'est pas occupé par le circuit d'audition électronique.

7. Dispositif selon l'une des revendications 1 à 4, **caractérisé en ce que** le corps occupe au moins 90% du volume du dispositif auditif qui est occupé par le circuit d'audition électronique.

8. Dispositif selon l'une des revendications 1 à 4, **caractérisé en ce que** le corps occupe au moins 99% du volume du dispositif auditif qui n'est pas occupé par le circuit d'audition électronique.

9. Dispositif selon l'une des revendications précédentes, **caractérisé en ce que** le corps n'est pas absorbant et est sensiblement imperméable au cérumène.

10. Dispositif selon l'une des revendications précédentes, **caractérisé en ce que** le corps abrite, au moyen d'encapsulation, le circuit auditif électronique de l'environnement hostile de l'oreille qui usuellement provoque la corrosion des connections exposées.

11. Dispositif selon l'une des revendications précédentes, **caractérisé en ce qu'**il constitue une aide auditive ayant un récepteur (5), un port récepteur, et une extrémité médiane, et le récepteur est en retrait par rapport à l'extrémité médiane de l'instrument, permettant ainsi au cérumène peut être extrudée du port récepteur lorsque la pression est exercé entre le récepteur extrémité médiane du dispositif.

12. Dispositif selon l'une des revendications précédentes, **caractérisé en ce que** le corps comporte un mélange d'élastomères et de particules conductrices, pourront réaliser un écran statique protégeant le circuit de RFI, GSM et EMI.

13. Dispositif selon l'une des revendications précédentes, **caractérisé en ce que** le circuit auditif électronique inclue un récepteur (5), un amplificateur (6), et un harnais de connexion reliant le récepteur est amplificateur, avec une boucle (26) en forme de S.

14. Dispositif selon l'une des revendications précédentes, **caractérisé en ce que** le circuit auditif électronique inclue des transducteurs et le corps, du fait de sa nature souple/solide, réalise une absorption d'échapper pour les transducteurs.

15. Dispositif selon l'une revendication précédentes, **caractérisé en ce qu'**il est réalisé à partir d'une impression standardisés de l'oreille, par moulage, ou à partir de différentes tailles choisis de l'oreille, afin de réaliser une plate-forme pour la fabrication d'une série de dispositif souple/solide, qui ne soit pas fait sur mesure.

16. Dispositif selon l'une revendication précédente de **caractérisé en ce que** le corps présente une valeur de dureté suffisamment basse Duromètre, Shore A, afin de permettre au dispositif d'être porté par l'utilisateur tout en dormant.

17. Dispositif selon l'une des revendications précédentes, **caractérisé en ce que** le circuit auditif électronique inclue une circuiterie d'aide à l'audition.

18. Dispositif selon l'une des revendications précédentes, **caractérisé en ce que** le dispositif est un dispositif du type disposé complètement dans le canal.

19. Dispositif selon l'une des revendications précédentes, **caractérisé en ce que** le corps est moulé sur mesure.

20. Dispositif selon la revendication 1, **caractérisé en ce que** au moins 90% du corps présente une dureté Duromètre, Shore A, de moins de 25 points.

21. Dispositif selon la revendication 1, **caractérisé en ce que** au moins 80% du corps présente une dureté Duromètre, Shore A, de moins de 35 points.

22. Dispositif selon la revendication 1, **caractérisé en ce que** le corps est un gel élastomère et présente une dureté Duromètre, Shore A, de moins de 35 points.

23. Dispositif selon la revendication 1, **caractérisé en ce que** le corps comporte un gel et présente une dureté Duromètre, Shore A, de moins de 35 points.

24. Dispositif selon la revendication 1, **caractérisé en ce qu'**il est constitué d'un dispositif d'audition complètement dans le canal, et **en ce que** :
(a) le corps du dispositif d'audition est moulé sur mesure ; et
(b) le circuit d'audition électronique comporte un réseau d'amplificateur incluant un circuit d'aide d'audition électronique, des transducteurs, et un contrôle de volume, le réseau d'amplification étant incorporé dans le corps.

25. Dispositif selon la revendication 1, **caractérisé en ce que** le dispositif (100) est un dispositif d'audition qui est porté à l'intérieur d'un canal auditif humain, et **en ce que** :
(a) le corps inclue un matériau de surface extérieure et un matériau de remplissage ;
(b) un circuit d'audition électronique comprenant un réseau d'amplification contenu dans le matériau de remplissage, le réseau d'amplification comprenant un circuit d'aide d'audition électronique, des transducteurs, et un contrôle de volume, et **en ce que** :
le matériau de surface extérieur et le matériau de remplissage présentent différentes valeurs de dureté, au moins l'un ayant une valeur de dureté Duromètre Hardness, Shore A, dans la gamme de 1 à 7 points, et
le matériau de remplissage en capsule le réseau d'amplification pour éliminer les espaces d'air qui autrement provoquerait un chemin de conduction air interne entre eux un récepteur (5) et un microphone (8) dans le réseau d'amplification.
